# EUROPÄISCHE PATENTSCHRIFT

(11) **EP 4 218 636 B1**
(45) Veröffentlichungstag und Bekanntmachung des Hinweises auf die Patenterteilung: **14.08.2024**
(21) Anmeldenummer: 22154210.3
(22) Anmeldetag: 31.01.2022
(51) Int. Cl.: A61B 17/88, A61F 2/36

(54) **ZEMENTAPPLIKATOR**
CEMENT APPLICATOR
APPLICATEUR DE CIMENT

(43) Veröffentlichungstag der Anmeldung: 02.08.2023
(73) Patentinhaber: Justus-Liebig-Universität Gießen, Körperschaft des öffentlichen Rechts, 35390 Gießen (DE)
(72) Erfinder: FÖLSCH, Christian, 35390 Gießen (DE); FONSECA-ULLOA, Carlos Alfonso, 35390 Gießen (DE); HARZ, Torben, 35392 Gießen (DE); JAHNKE, Alexander, 35633 Lahnau (DE)
(74) Vertreter: Stumpf, Peter

(56) Entgegenhaltungen:
- DE-A1- 102016 121 607
- US-A1- 2015 005 778
- US-A1- 2019 380 759

## Beschreibung

Die vorliegende Erfindung betrifft einen Zementapplikator zum Einbringen von Knochenzement bei der Implantation von Prothesen in Knochen.

### Beschreibung und Einleitung des allgemeinen Gebietes der Erfindung

Eine Implantation ist eine Operation zum Einsetzen eines künstlichen Materials in den Körper. Das eingesetzte Material, welches auch als Implantat oder als Prothese bezeichnet wird, verbleibt dabei permanent oder zumindest für einen längeren Zeitraum im Körper. Generell ist die Erhaltung der Anatomie und der Biomechanik ein fundamentales Ziel der Prothetik, insbesondere auch der Hüftendoprothetik. Dabei ist es gerade bei der Versorgung junger Patienten mit Gelenksersatz wichtig, möglichst knochenerhaltend und minimalinvasiv zu arbeiten. Schließlich wird durch die Erhaltung des Knochens die Verankerungsqualität bei einer nicht auszuschließenden späteren Wechseloperation verbessert. Diesem Anspruch versuchen immer mehr Hersteller durch die Entwicklung von Oberflächenersatzprothesen zur Therapie von Knochenarthrosen Rechnung zu tragen.

### Stand der Technik

Gegenwärtig wird bei der Implantation von Prothesen in Knochen, um z.B. durch Arthrose zerstörte Gelenke zu ersetzen (z.B. bei einer Knie-Totalarthroplastik), häufig Knochenzement verwendet, um das Implantat auf dem Knochen zu verfestigen. Mittlerweile werden Oberflächenersatz-Systeme weltweit von vielen Zentren implantiert. So wird bei Hüftimplantaten die femorale Fixation der Prothese zumeist zementiert durchgeführt, wobei die primäre Fixation von der Zementiertechnik abhängt.

Die Hauptaufgabe des Knochenzements besteht darin, Endoprothesen fest und sicher im Knochen zu verankern. An den künstlichen Gelenken können sehr große Kräfte auftreten. Zudem kann die Belastungssituation sehr komplex sein. Es gilt, Überlagerungen von Druck-, Scher-, Biege- und Torsionsbeanspruchung zu beachten. Der Zement muss die Prothese bereits unmittelbar nach dem Einsetzen des Implantats und anschließend über möglichst viele Jahre sicher im Knochen halten können.

In der zementierten Endoprothetik sind aseptische Lockerungen nach wie vorhäufigster Ursache für Revisionen. Dem Knochenzement als kraftübertragendem Material zwischen Prothese und Knochen kommt dabei eine kritische Rolle zu. Lufteinschlüsse schwächen den Zement und begünstigen Mikrofrakturen sowie deren Ausbreitung im Zementmantel. Mit der Vakuum-Mischtechnik können die Porosität des Knochenzements verringert und die mechanischen Eigenschaften verbessert werden.

Dabei zeigen Untersuchungen, dass die Rate der aseptischen Lockerungen der Prothesen, die zwischen Zement und Knochen und/oder zwischen Zement und Prothese auftreten, höher als bei konventionellen Langschaftprothesen ist und meist auf der femoralen Seite auftraten. So erfordert das Zement-Knochen-Interface ein suffizientes Eindringen des Zementes in die Spongiosa am proximalen Femur. Auch kann eine Benetzung dieses Bereiches des Knochens oder des Zements mit Blut nachteilige Effekte auf beide Interfaces haben. Zudem kann eine besonders tiefe Zementpenetration oder eine besonders dicke Zementschicht zu Hitzenekrosen im Knochen führen. So wurden in humanen Femora Temperaturen von über 50°C in 3 mm Abstand von der Zementoberfläche gemessen, die länger als 30 Sekunden andauerten und damit das Knochengewebe irreversibel schädigen konnten. Wenn hingegen zu viel Zement zwischen Knochen und Prothese verbleibt, resultiert daraus ein schlechter Prothesensitz, der mit biomechanisch ungünstigen Hebelverhältnissen und einem erhöhten Risiko für Schenkelhalsbrüche einhergeht. Zuviel Zement unter einer Oberflächenersatzprothese, im Knochen oder auf der Knochenoberfläche kann Knochennekrosen, Prothesenlockerungen und Schenkelhalsbrüche verursachen.

Im Allgemeinen besteht der Knochenzement aus einem Pulver aus Polymethylmethacrylat (PMMA) und einer Flüssigkeit aus Methylmethacrylat (MMA). Wenn PMMA und MMA in einem groben Verhältnis von 2:1 gemischt werden, bildet sich ein Knochenzement aus Polymethylmethacrylat. Sobald PMMA und MMA in Kontakt kommen, beginnt die Polymerisation. Zum Beispiel härtet der Knochenzement innerhalb eines Zeitintervalls von 7-15 Minuten aus und härtet vollständig aus. Je nach Vorliebe tragen die Chirurgen den Knochenzement früh innerhalb dieses Zeitintervalls auf, wenn der Knochenzement weniger viskos und flüssig ist, oder spät Knochenzement innerhalb dieses Zeitintervalls, wenn der Knochenzement zähflüssiger und kittartiger ist. Das Arbeiten mit einem weniger viskosen Knochenzement ist vorteilhaft, da der Knochenzement tiefer in den resezierten Knochen eindringen kann, was eine tiefer im Knochen verankerte Fixierung ermöglicht. Allerdings ist das Auftragen eines weniger viskosen Knochenzements deutlich schwieriger. Die Chirurgen haben nur sehr wenig Zeit, den Knochenzement innerhalb des kleinen Zeitrahmens gleichmäßig aufzutragen, wenn der Knochenzement seine optimale Konsistenz hat oder weniger viskos ist. Daher besteht auf dem Gebiet ein Bedarf an einem Zementapplikator, um die Effizienz des Auftragens des Knochenzements auf den Knochen eines Patienten zu verbessern.

Oftmals werden Zementapplikationen von den Chirurgen per Hand durchgeführt - eine Standardisierung ist hierdurch nicht möglich und unterliegt lediglich der entsprechenden Expertise des Operateurs. Durch eine individuelle Zementapplikation kann es zu starken Schwankungen insbesondere bei der Schichtdicke des Zements auf dem Knochen bei der Zementapplikation kommen - hierdurch kann die Zementpenetration in den Knochen und die damit verbundene Stabilität des Implantats stark beeinträchtigt werden.

Dazu wurden bereits verschiedene Hilfswerkzeuge und Applikatoren entwickelt.

Die Schrift US2015005778A1 offenbart einen Zementapplikator zum Auftragen eines Knochenzements auf eine Knochenoberfläche. Hier hängt die gleichmäßige Schichtdicke stark von der Erfahrung des Operateurs ab und wird nicht durch den Zementapplikator allein sichergestellt.

Die Schrift DE102007024083A1 offenbart einen alternativen Zementapplikator. Diese Vorrichtung dient zum Formen einer auf einem Knochen aufgebrachten Zementmasse. Der in dieser Schrift beschriebene Applikator umfasst keine Bestandteile um das Auftragen des Zements zu erleichtern oder den Zufluss an Zement zu regulieren. Damit kann auch dieses System keine Lösung zur Sicherstellung einer gleichmäßige Schichtdicke bereitstellen.

Diese bislang bekannten Vorrichtungen versetzen den Operateur nicht in die Lage, den Zement kontrolliert, gleichmäßig und glatt auf dem Knochen zu verteilen, um eine definierte Zementpenetration in den Knochen zu bewirken. Dabei treten regelmäßig folgende Probleme auf: Zum einen kann es zum Verschließen der für das Befestigen der Prothese vorgesehenen zentralen Öffnung und damit zu Lufteinschlüssen unter der Prothesenkappe kommen. Zum anderen kann zu wenig Zement am äußeren unteren Rand oder zu viel Zement am Prothesenpol unter der Prothesenkappe zu einem inkompletten Prothesensitz führen. Zudem kann es zu Blutlamination und Verunreinigungen durch Handkontakt kommen.

### Aufgabe

Aufgabe der vorliegenden Erfindung ist es, die Nachteile des Standes der Technik zu vermeiden, sodass ein Zementapplikator zur Erzeugung einer besonders gleichmäßigen Schicht von Knochenzement auf einem Knochen bereitgestellt werden kann, sodass eine besonders zuverlässige Verbindung zwischen Knochen und implantierter Prothese sichergestellt wird.

### Lösung der Aufgabe

Gelöst wird diese Aufgabe durch die kennzeichnenden Merkmale des Anspruchs 1. Weitere vorteilhafte Ausgestaltungen ergeben sich aus den Unteransprüchen.

Die Erfindung betrifft einen verbesserten Zementapplikator 100 zur Applikation von Knochenzement bei der Implantation von Prothesen.

Dazu wird der Zementapplikator 100 eingesetzt, um Zement von einem Mischsystem in welchem sich der Zement vor seiner Verwendung befindet, auf die

Oberfläche eines Knochens zu applizieren, damit anschließend eine Prothese auf dieser Oberfläche sicher verankert werden kann. Das Mischsystem, welches nicht Teil des eigentlichen Zementapplikators 100 ist, umfasst üblicherweise eine Knochenzementspritze 1 um den Knochenzement in einen Zementapplikator zu übertragen. Die Knochenzementspritze 1 weist normalweise eine Öffnung auf, durch welche der Knochenzement aus dem Mischsystem in einen Zementapplikator 100 austreten kann.

Der Zementapplikator 100 umfasst wenigstens einen Universalanschluss 3 zum Anschluss an ein Mischsystem. Dieser Universalanschluss 3 ist dabei so ausgebildet, dass er mit einem Mischsystem verbindbar ist, sodass Knochenzement 2 aus einem Mischsystem durch den Universalanschluss 3 gepresst werden kann. Der Durchmesser des Universalanschlusses weist dabei bevorzugt einen Durchmesser zwischen 2 mm und 10 mm auf, sodass er für die meisten üblichen Mischsysteme geeignet ist. Der Universalanschluss ist dabei vorzugsweise so ausgebildet, dass der Zementapplikator entweder per Presssitz aufgedrückt, oder über ein bestehende Außengewinde des Düsenschraubaufsatzes des Mischsystems aufgeschraubt werden kann.

Weiterhin umfasst der Zementapplikator 100 eine Vorkammer 11 aufweisend eine erste Seite 11a, eine zweite Seite 11b und einen Mantel 11c, der die ersten Seite 11a und die zweiten Seite 11b fluiddicht miteinander verbindet. Der Mantel 11c ist so ausgebildet, dass er das Innere der Vorkammer 11 dabei umschließt. Die Vorkammer 11 ist auf ihrer ersten Seite 11a mit dem Universalanschluss 3 fluiddurchströmbar verbunden und dient zu Aufnahme von Knochenzement aus einem Mischsystem.

Zusätzlich umfasst die Vorkammer 11 auf der dem Universalanschluss 3 gegenüberliegenden zweiten Seite 11b wenigstens ein Austrittloch 5. Je nach Größe des finalen Implantats umfasst die Vorkammer 11 vorzugsweise zwischen 20 und 30 Austrittslöcher 5 mit einem Durchmesser zwischen 1 mm und 5 mm. In einer alternativen Ausführungsform sind die Austrittslöcher 5 schlitzförmig mit einer Länge von 2 cm bis 4 cm und einer breite zwischen 1 mm und 3 mm ausgeführt. Dabei weist die Vorkammer vorzugsweise zwischen 12 und 24 schlitzförmige Austrittslöcher 5 auf, durch welche der Knochenzement 2 aus der Vorkammer 11 gepresst werden kann.

Weiterhin umfasst der Zementapplikator 100 einen Führungsdorn 6. Dieser ist an der zweiten Seite 11b der Vorkammer 11 so angeordnet, dass er mit einem ersten Ende 6a zentral in die Vorkammer 11 hineinragt und mit einem entgegengesetzten zweiten Ende 6b innerhalb eines präparierten Markraumkanals eines Knochens anbringbar ist.

Der Führungsdorn 6 ist mit einem ersten Ende 6a vorzugweise kegelförmig ausgebildet und soll somit ein gleichmäßiges Einströmen des Fluids in die Vorkammer 11 gewährleisten. Damit ist er so ausgebildet, dass er den Verschluss eines Mischsystems durchstoßen kann, sodass Knochenzement aus dem Mischsystem über den Universalanschluss 3 in die Vorkammer 11 gelangen kann.

In einer alternativen Ausführungsform ist der Führungsdorn 6 vorzugsweise fluiddurchströmbar ausgebildet und umfasst wenigstens ein Austrittsloch 14, sodass Knochenzement aus dem Mischsystem über den Universalanschluss 3 durch den Führungsdorn 6 über das wenigstens eine Austrittsloch 14 direkt in einen präparierten Markraumkanals gelangen kann. Das wenigstens eine Austrittsloch 14 ist dabei am zweiten Ende 6b angeordnet. Dabei umfasst der Führungsdorn vorzugsweise zwischen 10 und 20 Austrittslöcher 14 mit einer Größe von 0,5 mm bis 2 mm.

Die genaue Geometrie des Führungsdorns 6 ist abhängig vom Implantat und der Implantatgröße. Vorzugsweise weisen dieser Führungsdorn bei Knieendoprothesen eine zylindrische Geometrie auf. Das Material ist vorzugsweise ein druckbarer Kunststoff, welcher werkseitig sterilisiert und steril verpackt werden kann. Dies ist beispielsweise Polyetheretherketon (PEEK).

Der Zementapplikator 100 umfasst weiterhin wenigstens einen Austrittsbereich 9. Dieser ist über die zweite Seite 11b der Vorkammer mit der Vorkammer 11 verbunden und über wenigstens ein Austrittsloch 5 mit der Vorkammer 11 fluiddurchströmbar verbunden. Dabei bildet die zweite Seite 11b der Vorkammer 11 die erste Seite 9a des Austrittsbereichs 9. Weiterhin ist der Austrittsbereich 9 so ausgebildet, dass die mit ihrer der Vorkammer 11 gegenüberliegenden Seite offen und auf einem Knochen 7 anbringbar ist. So kann Knochenzement, von der Vorkammer 11 durch den Austrittsbereich 9 auf einem Knochen gleichmäßig aufgebracht werden.

Der Applikator ist so aufgebaut, dass ein Operateur nach der Zementapplikation den Zementapplikator 100 entfernen und ein Implantat in den Knochenzement pressen kann.

Dazu umfasst der Zementapplikator 100 wenigstens eine Torsionsschutzführung 8, sodass der flächig auf einem Knochen aufliegen kann. Diese ist dabei zwischen der Vorkammer 11 und dem Austrittsbereich 9 angeordnet. Die Torsionsschutzführung 8 ist speichenförmig vom Führungsdorn 6 ausgehend angeordnet. Diese kann dabei entweder einstückig mit dem Führungsdorn 6 oder separat ausgebildet sein. Die Torsionsschutzführung 8, übernimmt bei der Aufbringung des Knochenzements zwei Aufgaben. Die Torsionsschutzführung wird bei der Knochenzementapplikation in die bereits präparierten Schlitze im spongiösen Knochen eingeführt und verhindert somit ein verdrehen des Applikators um die longitudinale Achse des Knochens. Weiterhin soll bei der Zementapplikation kein Zement in diese Führungen gelangen. Durch einen passgenauen Sitz der Torsionsschutzführungen in den präparierten Schlitz, wirkt dieser zusätzlich abdichtend und verhindert somit das ungewollte Eindringen des Knochenzementes.

In einer weiteren alternativen Ausführungsform weist der Austrittsbereich 9 eine sie umgegebene Dichtung 10 auf. Diese ist dabei so angeordnet, dass sie sich zwischen der Vorkammer 11 und dem Austrittsbereich 9 auf der dem Führungsdorn 6 abgewandten Seite des Mantels 11c befindet. Die Dichtung 10 ist dabei als Dichtlippe oder Dichtwulst ausgebildet und dient zur Verringerung der Kontaktfläche zwischen Austrittsbereich und Knochen. Hierdurch wird ein konstanter Druck innerhalb des Zements gewährleitet. Mit eben diesem gleichmäßigen Druck kann Knochenzement an jeder Stelle der Knochenoberfläche zeitgleich flächig eingebracht werden.

In einer weiteren alternativen Ausführungsform umfasst der Zementapplikator 100 einen Druckregulierungswulst 12. Dieser ist vorzugsweise als Dichtungsring ausgeführt. Er ist ringförmig um den Führungsdorn 6 angeordnet und ragt dabei in die Vorkammer 11 hinein. Der Druckregulierungswulst 12 kann dabei entweder einstückig mit dem Führungsdorn 6 oder separat ausgebildet sein. Über die Dichtung 10 bzw. den Dichtungswulst 12 kann im Austrittsbereich 9 des Applikators die Dicke des Zementmantels auf dem Knochenplateau beeinflusst bzw. eingestellt werden.

In einer weiteren Ausführungsform umfasst der Zementapplikator 100 noch wenigstens eine Stabilisierungsrippe 13. Diese ist auf dem Mantel 11c der Vorkammer angeordnet und dabei vom Führungsdorn 6 ausgehend zum Rand des Mantels 11c ausgerichtet. Sie dient dazu die Steifigkeit des Zementapplikator zu erhöhen. Alternativ kann dies erreicht werden, wenn die Materialdicke des Mantels 11c entsprechend erhöht wird.

Die zusätzlichen Bauelemente der weiteren Ausführungsformen: wenigstens ein Austrittsloch 14, Dichtung 10, Druckregulierungswulst 12 und wenigstens eine Stabilisierungsrippe 13 sind miteinander kompatibel. So kann jedes dieser Bauelemente hinzugefügt oder wegelassen werden ohne die anderen Komponenten in ihrer Funktion zu beeinträchtigen. Welches Bauelement tatsächlich verwendet wird, hängt von der Art und der Größe des Implantates ab, für welches der Zementapplikator verwendet werden soll.

### Verwendung

Für die Verwendung des erfindungsgemäßen Zementapplikators 100 wird dieser zunächst an das verwendete Mischsystem über einen Universalanschluss 3 gesteckt, sodass der Zement 2 direkt hierdurch gespritzt werden kann. Durch einen internen zur Flussregulierung dienenden Führungsdorn 6 wird der Zement zunächst im Vorraum 11 gleichmäßig verteilt und tritt anschließend gleichmäßig über wenigstens ein unten liegendes Austrittsloch 5 aus. Hierdurch wird ein konstanter Druck des Knochenzements 2 gewährleitet. Mit eben diesem gleichmäßigen Druck wird der Knochenzement zeitgleich und flächig in den Knochen eingebracht. Über einen implantatindividuellen Führungsdorn 6 und eine Torsionsschutzführung 8 innerhalb des präparierten Markraumkanals des Knochens 7 wird zudem gewährleistet, dass der Zementapplikator 100 flächig auf dem Knochen 7 aufliegt. Nach der Zementapplikation kann der Operateur den Zementapplikator 100 entfernen und das Implantat in den Zement pressen. Über einen Dichtungsring bzw. einen Dichtungswulst kann zudem im Austrittsbereich 9 des Applikators 100 die Dicke des Zementmantels auf dem Schienbeinplateau beeinflusst bzw. eingestellt werden. Dies führt zur Erzeugung einer besonders gleichmäßigen Schicht von Knochenzement auf einem Knochen, sodass eine besonders zuverlässige Verbindung zwischen einen Knochen und einer implantierten Prothese sichergestellt.

Der erfindungsgemäße Zementapplikator ist grundsätzlich für verschiedene Arten von Implantation geeignet bei denen Prothesen in Knochen mittels Knochenzement befestigt werden. Beispielhaft seien hier Operationen zum Ersetzen eines Hüftgelenks, eines Kniegelenks oder eines Schultergelenks genannt. Das Verfahren ist aber nicht darauf einschränkt. Dabei kann das Implantat aus verschiedensten Materialen oder Materialkombinationen bestehen. Beispielshafte Gruppen von Materialien sind Edelstähle, Titanverbindungen, Keramiken oder Kunststoffe. Dazu ist es notwendig den Zementapplikator an die jeweilige Gelenkgeometrie anzupassen.

### Ausführungsbeispiel

Eine beispielhafte Ausführung des Zementapplikators basiert auf der Geometrie der Tibiakomponente des P.F.C.^{™} SIGMAOTC3-Kniesystems (J&J Medical Devices). Nach intraoperativer Auswahl der finalen Größe, wird ein entsprechender Applikator ausgewählt, welcher der Geometrie und der Größe des zu verwendeten Implantats 1:1 entspricht - diese Passung wird dadurch realisiert, dass Hersteller, die einen solchen Applikator für die Implantation ihrer Implantate zur Verfügung stellen wollen, die 3D-Daten zur Verfügung stellen. Nach Präparation des Implantatlagers wird der Zement angerührt und anschließend das Mischsystem auf den Zementapplikator gesteckt/geschraubt. Der Führungsdorn wird in die präparierte Markhöhle eingeführt. Die Torsionsstellung wird in diesem konkreten Fall über die Torsionsschutzführung realisiert. Bei Implantaten, die keine Flügel aufweisen, wird eine mögliche Verdrehung des Implantats visuell kontrolliert. Abschließend wird der Zement durch das Mischsystem in den Applikator gepresst und verteilt sich über diesen gleichmäßig auf der präparierten Oberfläche des Kochens, die als Implantatlager dient.

### Abbildungslegenden und Bezugszeichenliste

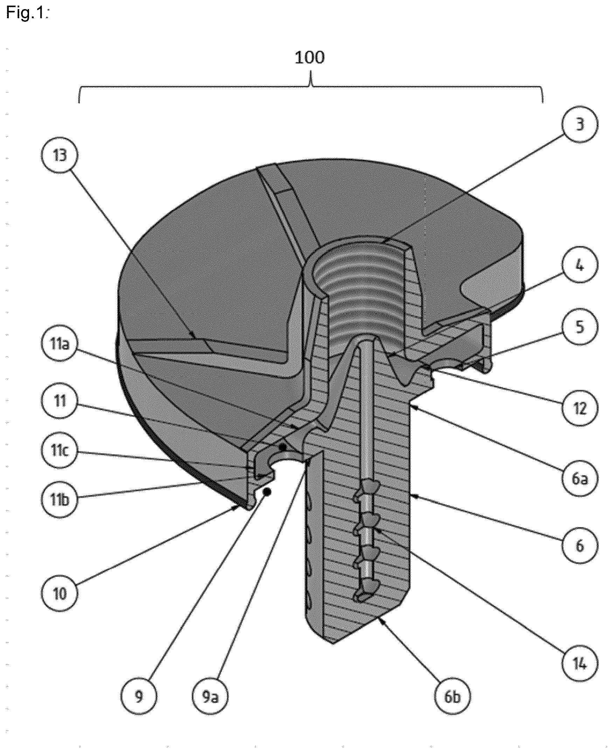

Fig. 1 zeigt eine beispielhafte Ausführungsform des erfindungsgemäßen Zementapplikators 100 in einer isometrischen Ansicht.

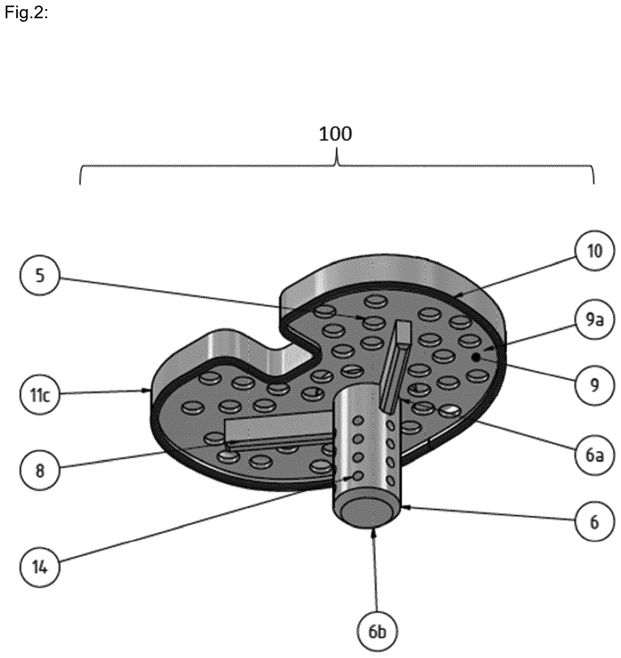

Fig. 2 zeigt eine beispielhafte Ausführungsform des erfindungsgemäßen Zementapplikators 100 im betriebsbereiten Zustand in einer isometrischen Ansicht. Hierbei ist der Zementapplikator 100 mit einem Mischsystem in Form einer Knochenzementspritze 1 mit Knochenzement 2 verbunden. Die Fig.2 zeigt den Zementapplikator 100 wie dieser bereits über den Führungsdorn 6 im Knochen verankert wurde, um den Knochenzement 2 auf die Oberfläche des Knochens 7 zu applizieren.

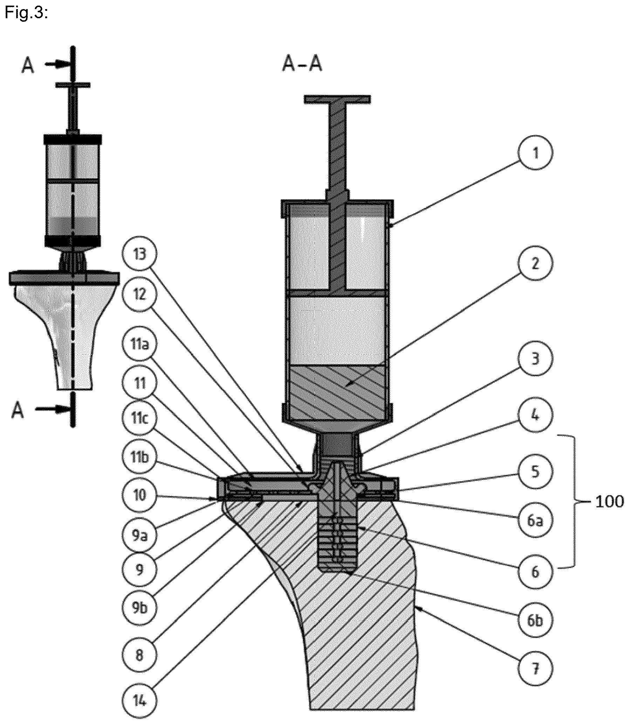

Fig. 3 zeigt eine beispielhafte Ausführungsform des erfindungsgemäßen Zementapplikators 100 zusammen mit einer mit Knochenzement 2 gefüllten Knochenzementspritze 1 und einem Knochen 7 auf dessen Oberfläche dieser Zement appliziert werden soll als Gesamtkonzept.

### Bezugszeichen

1 Knochenzementspritze
2 (Knochen-)zement
3 Universalanschluss
4 Flussregulierungsdorn
5 Austrittsloch der Vorkammer
6 Führungsdorn
6a erstes Ende des Führungsdorns
6b zweites Ende des Führungsdorns
7 Knochen
8 Torsionsschutzführung
9 Austrittsbereich
9a erste Seite des Austrittsbereichs
9b zweite Seite des Austrittsbereichs
10 Dichtung
11 Vorkammer
11a erste Seite der Vorkammer
11b zweite Seite der Vorkammer
11c Mantel der Vorkammer
12 Druckregulierungswulst
13 Stabilisierungsrippe
14 Austrittsloch des Führungsdorns
100 Zementapplikator

## Patentansprüche

1. Zementapplikator (100) zum Einbringen von Knochenzement bei der Implantation von Prothesen in Knochen wenigstens umfassend
• einen Universalanschluss (3) zum Anschluss an ein Mischsystem
• eine Vorkammer (11) aus einer ersten Seite (11a), einer zweiten Seite (11b) und einem Mantel (11c), der die erste Seite (11a) und die zweite Seite (11b) miteinander fluiddicht verbindet,
• wobei die erste Seite (11a) mit dem Universalanschluss (3) fluiddurchströmbar verbindbar ist und zur Aufnahme von Knochenzement aus einem Mischsystem geeignet ist, und
• wobei die zweite Seite (11b) der ersten Seite (11a) gegenüberliegend angeordnet ist und wenigstens ein Austrittloch (5) aufweist, durch welches der Knochenzement austreten kann,
• ein Austrittsbereich (9), welcher benachbart zur Vorkammer (11) angeordnet ist, wobei die zweite Seite (11b) der Vorkammer die erste Seite (9a) der Austrittsbereich (9) bildet, sodass die Austrittsbereich (9) über das wenigstens eine Austrittsloch (5) mit der Vorkammer (9) fluiddurchströmbar verbunden ist, sodass Knochenzement von der Vorkammer (11) in die Austrittsbereich (9) strömen kann, wobei der Austrittsbereich (9) weiterhin so ausgebildet ist, dass seine der Vorkammer (11) gegenüberliegende zweite Seite (9b) offen und auf einem Knochen anordbar ist,
• eine Torsionsschutzführung (8), wobei diese zwischen der Vorkammer (11) und der Austrittkammer (9) angeordnet ist, sodass der Zementapplikator (100) flächig auf einem Knochen aufliegt kann
**dadurch gekennzeichnet, dass** der Zementapplikator(100) weiterhin einen Führungsdorn (6) aufweist, welcher so angeordnet ist, dass er mit einem ersten Ende (6a) so in die Vorkammer (11) hineinragt, sodass er durch wenigstens einen Universalanschluss (3) hindurch einen Verschluss eines Mischsystems durchstoßen kann, sodass Knochenzement aus dem Mischsystem über den Universalanschluss (3) in die Vorkammer (11) gelangen kann, wobei ein dem ersten Ende entgegensetztes zweites Ende (6b) des Führungsdorns (6), innerhalb eines präparierten Markraumkanals eines Knochens anbringbar ist.

2. Zementapplikator (100) gemäß Anspruch 1 **dadurch gekennzeichnet, dass** der Führungsdorn (6) durchströmbar ausgebildet ist und wenigstens ein Austrittsloch (14) aufweist, sodass Knochenzement aus dem Mischsystem über den Universalanschluss (3) durch den Führungsdorn (6) über das wenigstens eine Austrittsloch (14) direkt in einen präparierten Markraumkanals gelangen kann.

3. Zementapplikator (100) gemäß Anspruch 1 oder 2 **dadurch gekennzeichnet, dass** der Austrittsbereich (9) eine sie umgegebene Dichtung (10) aufweist, welche. so angeordnet ist, dass sie sich zwischen der Vorkammer (11) und dem Austrittsbereich (9) auf der dem Führungsdorn (6) abgewandten Seite des Mantels (11c) der Vorkammer befindet.

4. Zementapplikator (100) gemäß einem der vorigen Ansprüche **dadurch gekennzeichnet, dass** der Zementapplikator (100) einen Druckregulierungswulst (12) aufweist, welcher ringförmig um den Führungsdorn (6) angeordnet ist und dabei in die Vorkammer (11) hineinragt.

5. Zementapplikator (100) gemäß einem der vorigen Ansprüche **dadurch gekennzeichnet, dass** er noch wenigstens eine Stabilisierungsrippe (13) umfasst, wobei diese auf dem Mantel (11c) der Vorkammer angeordnet ist und dabei vom Führungsdorn (6) ausgehend zum Rand des Mantels (11c) ausgerichtet ist.

## Claims

1. Cement applicator (100) for introducing bone cement during the implantation of prostheses in bone, comprising at least
- a universal connection (3) for connection to a mixing system
- a prechamber (11) comprising a first side (11a), a second side (11b) and a jacket (11c) which connects the first side (11a) and the second side (11b) to one another in a fluid-tight manner,
- wherein the first side (11a) can be connected to the universal connection (3) through which fluid can flow and is suitable for receiving bone cement from a mixing system, and
- wherein the second side (11b) is arranged opposite the first side (11a) and has at least one outlet hole (5) through which the bone cement can emerge,
- an outlet region (9), which is arranged adjacent to the prechamber (11), wherein the second side (11b) of the prechamber forms the first side (9a) of the outlet region (9), so that the outlet region (9) is connected to the prechamber (9) via the at least one outlet hole (5) so that fluid can flow through it, so that bone cement can flow from the prechamber (11) into the outlet region (9), wherein the outlet region (9) is further designed such that its second side (9b) opposite the prechamber (11) is open and can be arranged on a bone,
- a torsion protection guide (8), wherein this is arranged between the prechamber (11) and the outlet chamber (9), so that the cement applicator (100) can rest flat on a bone
**characterised in that** the cement applicator (100) further comprises a guide mandrel (6) which is arranged such that it projects with a first end (6a) into the prechamber (11) such that it can pierce a closure of a mixing system through at least one universal connection (3), so that bone cement can pass from the mixing system via the universal connection (3) into the antechamber (11), wherein a second end (6b) of the guide mandrel (6) opposite the first end can be attached within a prepared medullary canal of a bone.

2. Cement applicator (100) according to claim 1, **characterised in that** the guide mandrel (6) is designed to allow flow through it and has at least one outlet hole (14), so that bone cement can pass from the mixing system via the universal connection (3) through the guide mandrel (6) via the at least one outlet hole (14) directly into a prepared medullary canal.

3. Cement applicator (100) according to claim 1 or 2, **characterised in that** the outlet region (9) has a seal (10) surrounding it, which is arranged in such a way that it is located between the prechamber (11) and the outlet region (9) on the side of the casing (11c) of the prechamber facing away from the guide mandrel (6).

4. Cement applicator (100) according to one of the preceding claims, **characterised in that** the cement applicator (100) has a pressure-regulating bead (12) which is arranged annularly around the guide mandrel (6) and thereby projects into the prechamber (11).

5. Cement applicator (100) according to one of the preceding claims, **characterised in that it** also comprises at least one stabilising rib (13), wherein this is arranged on the casing (11c) of the prechamber and, starting from the guide mandrel (6), is aligned towards the edge of the casing (11c.

## Revendications

1. applicateur de ciment (100) pour l'introduction de ciment osseux lors de l'implantation de prothèses dans l'os, comprenant au moins
- un raccord universel (3) pour le raccordement à un système de mélange
- une préchambre (11) constituée d'un premier côté (11a), d'un deuxième côté (11b) et d'une enveloppe (11c) qui relie le premier côté (11a) et le deuxième côté (11b) de manière étanche aux fluides,
- le premier côté (11a) pouvant être relié au raccord universel (3) de manière à pouvoir être traversé par un fluide et étant adapté pour recevoir du ciment osseux provenant d'un système mixte, et
- le deuxième côté (11b) étant disposé à l'opposé du premier côté (11a) et présentant au moins un trou de sortie (5) à travers lequel le ciment osseux peut sortir,
- une zone de sortie (9) qui est disposée à proximité de l'antichambre (11), le deuxième côté (11b) de l'antichambre formant le premier côté (9a) de la zone de sortie (9), de sorte que la zone de sortie (9) est reliée à l'antichambre (9) de manière à pouvoir être traversée par un fluide via le au moins un trou de sortie (5), de sorte que le ciment osseux peut s'écouler de la préchambre (11) dans la zone de sortie (9), la zone de sortie (9) étant en outre conçue de telle sorte que son deuxième côté (9b) opposé à la préchambre (11) est ouvert et peut être disposé sur un os,
- un guide de protection contre la torsion (8), celui-ci étant disposé entre la préchambre (11) et la chambre de sortie (9), de sorte que l'applicateur de ciment (100) peut reposer à plat sur un os.
**caractérisé en ce que** l'applicateur de ciment (100) présente en outre un mandrin de guidage (6) qui est disposé de telle sorte qu'il pénètre avec une première extrémité (6a) dans la préchambre (11) de telle sorte qu'il puisse percer une fermeture d'un système de mélange à travers au moins un raccord universel (3), de sorte que le ciment osseux peut passer du système de mélange dans l'antichambre (11) par l'intermédiaire du raccord universel (3), une deuxième extrémité (6b) du mandrin de guidage (6), opposée à la première extrémité, pouvant être placée à l'intérieur d'un canal médullaire préparé d'un os.

2. Applicateur de ciment (100) selon la revendication 1, **caractérisé en ce que** le mandrin de guidage (6) est conçu de manière à pouvoir être traversé par un écoulement et présente au moins un trou de sortie (14), de sorte que le ciment pour os peut parvenir du système de mélange via le raccord universel (3) à travers le mandrin de guidage (6) par le biais du au moins un trou de sortie (14) directement dans un canal médullaire préparé.

3. Applicateur de ciment (100) selon la revendication 1 ou 2, **caractérisé en ce que** la zone de sortie (9) comporte un joint d'étanchéité (10) qui l'entoure et qui est disposé de manière à se trouver entre la préchambre (11) et la zone de sortie (9), du côté de l'enveloppe (11c) de la préchambre qui est opposé au mandrin de guidage (6).

4. Applicateur de ciment (100) selon l'une des revendications précédentes, **caractérisé en ce que** l'applicateur de ciment (100) présente un bourrelet de régulation de pression (12) qui est disposé en forme d'anneau autour du mandrin de guidage (6) et qui fait alors saillie dans la préchambre (11).

5. Applicateur de ciment (100) selon l'une des revendications précédentes, **caractérisé en ce qu'**il comprend encore au moins une nervure de stabilisation (13), celle-ci étant disposée sur l'enveloppe (11c) de la préchambre et étant orientée en partant du mandrin de guidage (6) vers le bord de l'enveloppe (11c).
